# EUROPÄISCHE PATENTSCHRIFT

(11) **EP 1 266 977 B1**
(45) Veröffentlichungstag und Bekanntmachung des Hinweises auf die Patenterteilung: **25.05.2005**
(21) Anmeldenummer: 02019340.5
(22) Anmeldetag: 31.01.2001
(51) Int. Cl.: C23C 14/00, C09C 1/24, C09C 1/00

(54) **Glanzpigment und Verfahren zu seiner Herstellung**
Lustrous pigment and method of producing the same
Pigment interférentiel et procédé de production

(30) Priorität: 04.02.2000 DE 10004888
(43) Veröffentlichungstag der Anmeldung: 18.12.2002
(62) Teilanmeldung aus: 01949009.3
(73) Patentinhaber: ECKART GmbH & Co. KG, 90763 Fürth (DE)
(72) Erfinder: Sommer, Günter, Dr., 91217 Hersbruck (DE)
(74) Vertreter: Schneck, Herbert, Dipl.-Phys., Dr.

(56) Entgegenhaltungen:
- WO-A-93/08237
- WO-A-98/38254
- US-A- 3 123 490
- US-A- 5 135 812
- PATENT ABSTRACTS OF JAPAN vol. 013, no. 249 (C-605), 9. Juni 1989 (1989-06-09) & JP 01 054071 A (NISSHIN STEEL CO LTD), 1. März 1989 (1989-03-01)

## Beschreibung

Die Erfindung richtet sich auf ein Verfahren zur Herstellung eines Glanzpigments. Je nach Schichtaufbau können bei derartigen Pigmenten Interferenzphänomene auftreten. Solche Glanzpigmente sind beispielsweise aus US-A-3,123,490 oder WO 98 38 254 bekannt.

Glanzpigmente finden vielfache Anwendung, insbesondere für dekorative Zwecke, Automobillackierungen, kosmetische Zwecke oder im Bereich des Sicherheitsdrucks.

Bei bekannten Herstellungsverfahren wird auf die Oberfläche der Unterlage zunächst ein anorganisches Metallsalz, z.B. Natriumchlorid, als das spätere Ablösen von der Unterlage ermöglichende Zwischenschicht aufgebracht, wobei nachfolgend im CVD- oder PVD-Verfahren mehrere Schichten aufgedampft werden, und wobei der so gebildete Schichtenverbund nach dem Verlassen des Vakuumbereichs durch Lösen der Zwischenschicht mit Wasser in Form einzelner Partikel abgelöst wird.

Eine Vorrichtung zur Durchführung eines solchen Verfahrens, bei welcher als Träger ein umlaufendes Metallband verwendet wird, ist aus der nicht vorveröffentlichten deutschen Patentanmeldung 199 02 141 bekannt. Es wird dort weiterhin beschrieben, eine Reflexionsschicht bzw. Trägerschicht aus Aluminium durch Aufdampfen herzustellen, auf welche anschließend eine transparente Schicht, beispielsweise aus Magnesiumfluorid oder Titanoxid, aufgedampft wird.

Gemäß DE 1 242 778 ist vorgesehen, dass die aufgedampften Schichten aus der Gruppe Zinksulfid, Zinkoxid, Guanin, Magnesiumfluorid, Titandioxid, Calciumfluorid und Kryolit bestehen, wobei als Trägersubstanz für diese Filme ein Stoff aus der Gruppe der Alkalihalogenide, Erdalkalihalogenide oder Alkaliborate, wie z.B. Na₂B₄O₇; B₂O₃; MgCI₂ verwendet wird.

Die Verdampfungstemperaturen hängen von der jeweils pigmentbildenden Substanz bzw. Trägersubstanz ab.

Zur Farbenbildung werden Interferenzerscheinungen eingesetzt, indem man den Gangunterschied der Lichtwellen bedingt durch die Reflexion an den durch die verschiedenen aufgedampften Schichten erzeugten Grenzflächen ausnutzt. Diese Interferenzeffekte werden beeinflußt durch die Dicke der einzelnen Schichten und deren Brechungsindex. Für ein ungefärbtes perlmuttartiges Pigment soll beispielsweise das Produkt aus Filmdicke in nm und Brechzahl in einem Bereich zwischen 10 und 200 bleiben.

Interferenzfarben werden hervorgerufen, wenn der Wert dieses Produkts in den Bereich über 200 fällt, wobei die kräftigsten Farben bei einer gegebenen gewichtsmäßigen Pigmentkonzentration im Bereich von 200 bis etwa 1500 auftreten. Die Farbintensität hängt auch von der Gleichmäßigkeit der Plättchendicke ab, d.h. von der Planparallelität des Trägermaterials und der Planparallelität der aufgedampften Schichten, da Pigmentplättchen ungleichmäßiger Dicke unterschiedliche Farben reflektieren, welche sich gegenseitig aufheben können, sodass ein Farbeffekt nicht eintritt oder jedenfalls keine reinen Farben erzeugt werden.

Eine zusammenfassende aktuelle Darstellung des Standes der Technik findet sich in Chem. Rev. 1999, 99, S. 1963 - 1981. Dort wird aufgezeigt, dass die maximale Reflexion sich zu kürzeren Wellenlängen verlagert, wenn der Blickwinkel vergrößert wird. Dementsprechend kann ein mit weißem Licht beleuchteter dünner Film wechselnde Farben über das gesamte Spektrum des sichtbaren Lichts von Rot bis Blau zeigen, wenn der Beobachter seinen Blickwinkel in Richtung auf flache Winkel ändert. Es wird auch mathematisch aufgezeigt, dass eine größere Dickenstreuung zu weniger definierten Farben führt. Dies ist beispielsweise bei natürlichem Glimmer der Fall, wenn dieser als Substrat verwendet wird. Dementsprechend zeigen einzelne Partikel Interferenzerscheinungen, nicht aber eine Anordnung mehrerer Partikel. Die optischen Charakteristika einer solchen Anordnung basierend auf natürlichem Glimmer sind fast die gleichen wie diejenigen einer einzelnen Metalloxidschicht, insbesondere TiO₂-Schicht. Demgegenüber weisen neuere Perlglanzpigmente auf Basis von künstlich hergestellten Aluminiumoxid- und Siliciumoxidsubstraten eine konstante Dicke auf und werden damit Teil des optischen Systems. Mehrschicht-Oxid-Beschichtungen auf der Basis von SiO₂-Substraten zeigen stärkere und klarere Interferenzfarben als bei der Verwendung von Glimmer als Substrat.

Die Abscheidung aus der Flüssigphase oder aus der Gasphase führt zu einer signifikanten Porosität von oft mehr als 25% der Schicht. Durch diese Porosität wird die Intensität des reflektierten Lichts geschwächt.

Für die Herstellung anorganischer Filme wird das Vakuumaufdampfen, auch mit Elektronenstrahlen, das Sputtern und das CVD-Verfahren als an sich bekannt beschrieben. Diese Verfahren seien aber sehr kostenaufwendig und würden deshalb nur für optische Linsen, Filter und dergleichen eingesetzt.

Zur Herstellung von TiO₂-Flocken sei es bekannt, kontinuierliche Filme aufzubrechen, welche beispielsweise durch thermische Hydrolyse von Ti-OCl₂ hergestellt werden. Alternative Möglichkeiten bestehen in der Aufbringung von Titan-Alkoxiden auf eine Oberfläche und im Aufbrechen des resultierenden Films durch Dampfbehandlung, dem Aufbringen von kolloidaler TiO₂-Lösung auf eine Glasoberfläche und dem Abkratzen des resultierenden Films. Beschrieben wird auch die Aufbringung von TiOCl₂-Lösung auf einen Gelantinefilm und Ablösen des Gelantinefilms, Vakuumablagerung, Behandlung von Kaliumtitanat-Fasern mit Säure und nachfolgendes Erhitzen oder Herstellung und Aufbrechen hohler TiO₂-Teilchen aus der Mischung eines Tensids mit einer kolloidalen TiO₂-Lösung. Substratfreie TiO₂-Flocken können auch dadurch erhalten werden, dass man die Substrate in starken Säuren oder Basen löst.

Bei der Herstellung von TiO₂-beschichtetem Glimmer aus einer TiOCl₂-Lösung muß stark auf die Reaktorgeometrie und die Mischbedingungen geachtet werden. Die erhaltenen TiO₂-Schichten können mit organischen Farbstoffen beschichtet werden ebenso wie mit dünnen Schichten von Silber, Nickel oder Mischungen verschiedener Metalle, um einen dunkleren Ton zu erzeugen.

Aus DE-AS 1 136 042 ist es bekannt, Trägersubstanzen mit niedrigem Brechungsindex in Form von Oxiden oder Oxidhydraten von Metallen der IV. und/oder V. Gruppe, z.B. SiO₂, zu verwenden und diese mit Substanzen mit höherem Brechungsindex, z.B. Oxiden von Ti, Fe, Sb, Sn usw. zu beschichten.

Die Herstellung der dort beschriebenen Plättchen wird so bewerkstelligt, dass zunächst ein Träger aus Glas, keramischer Masse, Metall oder Kunststoff mit einer Lösung einer hydrolisierbaren Verbindung des in das gewünschte Oxid umzuwandelnden Metalls benetzt wird, wobei aus dem so erzeugten Flüssigkeitsfilm durch anschließendes Erhitzen das jeweilige Oxid oder Oxidhydrat als dünner Überzug gebildet wird.

Aus US 3,438,796 ist es bekannt, Pigmentplättchen herzustellen, die durch eine Mehrzahl von Filmen aus Siliciumoxid und Aluminium gebildet sind, wobei der Siliciumfilm einerseits als Schutzschicht für das Aluminium und andererseits zur Erzeugung von Interferenzeffekten in Abhängigkeit von seiner Dicke dient. Der Aluminiumfilm kann in seiner Dicke so eingestellt werden, dass er im wesentlichen lichtundurchlässig ist. Die Schichten werden durch chargenweises Aufdampfen erzeugt.

Gemäß EP 0 803 549 ist vorgesehen, dass auf in konventioneller Weise hergestellte plättchenförmige Partikel mit einer Länge von beispielsweise 1 bis 200 µm Siliciumoxidschichten aufgedampft werden. Die Plättchen sollen dabei aus metallisch reflektierendem Material oder Metallegierungen bestehen. Alternativ wird die Verwendung von Glimmer in Betracht gezogen. Dort wird weiterhin beschrieben, dass es bekannt ist, auf eine derartige Trägersubstanz aus einem farblosen Oxid Schichten aus Titanoxid in kristallisierter Form, z.B. als Rutil, aufzubringen. Hierauf kann gegebenenfalls eine weitere Schicht aus einem farbigen anorganischen Stoff, wie Eisenoxid, aufgebracht werden.

Aus früheren Untersuchungen ist es weiterhin bekannt, dass Brechzahl und Absorption von aufgedampften Siliciumoxidschichten um so niedriger werden, je langsamer die Verdampfung erfolgt und je größer der Sauerstoffgehalt ist, sodass so die Möglichkeit besteht, beim Aufdampfprozeß Einfluß auf die optischen Eigenschaften zu nehmen.

Alle vorstehend beschriebenen Pigmente sind gegebenenfalls durch Nachbehandlung oder Nachbeschichtung gegen Witterungseinflüsse und gegen Licht stabilisiert.

Hiervon ausgehend liegt der Erfindung die Aufgabe zugrunde, ein Verfahren zur Herstellung eines Glanzpigmentes bzw. ein Glanzpigment zu schaffen, welches mit einer hohen Produktionsrate herstellbar ist, sich durch sehr gute Haltbarkeitseigenschaften und durch eine breite Palette möglicher Farbtöne bei hoher Farbsättigung und Deckkraft auszeichnet.

Diese Aufgabe wird erfindungsgemäß dadurch gelöst, dass zunächst eine Trägerschicht durch Vakuum-Bedampfung eines umlaufenden Metallbandes hergestellt und nach Ablösen von der Unterlage die so erzeugten Trägerschicht-Partikel auf eine gewünschte Größe zerkleinert werden, wobei anschließend diese Trägerschicht-Partikel durch Naßbeschichtung mit wenigstens einer weiteren Beschichtung versehen werden.

Die erfindungsgemäße Trägerschicht weist durch ihre einfache Herstellung auch Vorteile gegenüber synthetischem Glimmer (Phlogopit) auf, der wegen seiner aufwendigen Herstellung bei hohem Druck und hoher Temperatur ohnehin niemals kommerzielle Bedeutung erlangt hat.

Durch die Aufdampfrate und den Sauerstoffpartialdruck können die optischen Eigenschaften wie Brechzahl und Reflexionskoeffizient der Trägerschicht eingestellt werden und es ist hierdurch möglich, definierte und reproduzierbare Farbtöne und Farbwechsel in Abhängigkeit vom Blickwinkel zu erzeugen.

Im Gegensatz zu naßchemisch beschichteten Perlglanzpigmenten sind erfindungsgemäß hergestellte Pigmente bis zur Außenkante hin planparallel.

Dies hat eine höhere Farbreinheit und einen besseren Glanz zur Folge, und zwar auch bei kleineren Teilchen, da der Streukantenanteil minimiert ist.

Es treten auch keine Nebenfällungen auf, die bei naßchemischen Verfahren zu beobachten sind. Weiterhin gibt es praktisch keine Kristallisationskeime als Ausgangspunkt für ein Kristallwachstum, da das Substrat eine sehr geringe Porosität und glatte Oberfläche aufweist.

Das erfindungsgemäße Herstellungsverfahren führt dazu, dass die vom Träger abgelösten Pigmentplättchen untereinander weitgehend identische und reproduzierbare optische Eigenschaften, z.B. den gleichen Farbton bei der Betrachtung aus einem bestimmten Blickwinkel, aufweisen.

Weiterhin fallen beim erfindungsgemäßen Verfahren keine Folienabfälle mit den entsprechenden kostenaufwendigen Entsorgungsproblemen an. Es ist erfindungsgemäß möglich, auch Substanzen, wie z.B. TiO, mit hoher Verdampfungstemperatur einzusetzen, was bei den herkömmlichen Verfahren unter Verwendung einer Kunststoff-Folie wegen der fehlenden Temperaturbeständigkeit dieser Folien nicht möglich wäre.

Sofern im Nachfolgenden von einer Schichtfolge die Rede ist, ist dies so zu verstehen, dass selbstverständlich jeweils auch die umgekehrte Schichtfolge geschützt sein soll, da nach dem Ablösen der Plättchen von der Unterlage die durch die Unterlage ursprünglich vorgegebene Orientierung nicht mehr gegeben ist.

Man nutzt bei dem erfindungsgemäßen Verfahren also insbesondere die vorstehend schon geschilderten grundsätzlichen Vorteile von Trägerschicht-Partikeln aus, die auf einer Metallunterlage abgeschieden werden, und kann die anschließende Beschichtung dann mit bekannten Naßbeschichtungs-Techniken realisieren. Die Trägerschicht kann dabei insbesondere aus Siliciumoxid, Silikat, Boroxid, Boraten, Aluminiumoxid, Aluminaten, Titanborid oder Gemischen hieraus bestehen.

Weiterhin kann vorgesehen sein, dass die Trägerschicht-Partikel Netzwerkbildner oder Netzwerkwandler und/oder Bariumsulfat zur Glättung der Oberfläche und/oder lösliche oder unlösliche anorganische oder organische Farbmittel umfassen.

Die naßchemisch aufgebrachten Schichten können vorteilhafterweise aus Oxiden der Metalle Zirkonium, Chrom, Titan, Eisen, Zink, Oxidhydraten dieser Metalle, Eisentitanaten, Titansuboxiden oder Mischungen hieraus bestehen, wobei die Metalloxide gegebenenfalls reduziert werden können. In an sich bekannter Weise können zusätzliche Beschichtungen zur Lichtund Wetterstabilisierung aufgebracht werden.

Um das Ablösen der Beschichtung von der Unterlage zu erleichtern, kann gegebenenfalls ein Releasecoat bestehend aus einem Lack, einem Salz, einer salzartigen Verbindung oder einem organischen Material aufgebracht werden. Die Abscheidung dieses Releasecoats kann beispielsweise durch Lackieren oder Verdampfen erfolgen.

Nachfolgend wird die Erfindung anhand eines Ausführungsbeispieles näher erläutert.

Auf ein umlaufendes Metallband wird unter Vakuum (10⁻⁴ mbar) zunächst eine Releaseschicht aus einem in Wasser löslichen, im Hochvakuum unzersetzt verdampfbaren Salz aufgedampft und anschließend eine Schicht aus SiO.

Die Umlaufgeschwindigkeit des Bandes ist 2 m/s, die Temperatur der SiO-Quelle 1450°C.

Die optische Dicke der SiO-Schicht beträgt 200 nm.

Nach dem Ablösen des Films vom Metallband in einem Wasserbad wird dieser durch Rühren mit einem Hochgeschwindigkeitsrührer zu Pigmentteilchen mit einer Größe von 1 bis 100 µm zerkleinert.

Das erhaltene Pulver wird nachfolgend naßchemisch in bekannter Weise mit Titandioxid beschichtet.

Je nach Schichtdicke werden Pigmente mit verschiedenen Interferenzfarben erhalten.

Die Messung der optischen Schichtdicke erfolgte nach der Schwingquarzmethode.

## Patentansprüche

1. Verfahren zur Herstellung eines Glanzpigments, **dadurch gekennzeichnet, dass** zunächst eine Trägerschicht durch Vakuum-Bedampfung eines umlaufenden Metallbandes hergestellt und nach Ablösen von der Unterlage die so erzeugten Trägerschicht-Partikel auf eine gewünschte Größe zerkleinert werden, wobei anschließend diese Trägerschicht-Partikel durch Naßbeschichtung mit wenigstens einer weiteren Beschichtung versehen werden.

2. Verfahren nach Anspruch 1, **dadurch gekennzeichnet, dass** die Trägerschicht-Partikel aus Siliciumoxid, Silikat, Boroxid, Boraten, Aluminiumoxid, Aluminaten, Titanborid (TiB₂) oder Gemischen hieraus besteht.

3. Verfahren nach Anspruch 1, **dadurch gekennzeichnet, dass** die Trägerschicht-Partikel Netzwerkbildner oder Netzwerkwandler und/oder Bariumsulfat zur Glättung der Oberfläche und/oder lösliche oder unlösliche anorganische oder organische Farbmittel umfassen.

4. Verfahren nach Anspruch 1, **dadurch gekennzeichnet, dass** die naßchemisch aufgebrachten Schichten aus Oxiden der Metalle Zirkonium, Chrom, Titan, Eisen, Zink, Oxidhydraten dieser Metalle, Eisentitanat, Titansuboxiden oder Mischungen hieraus bestehen.

5. Verfahren nach Anspruch 4, **dadurch gekennzeichnet, dass** die Metalloxide reduziert werden.

6. Verfahren nach Anspruch 1, **dadurch gekennzeichnet, dass** zusätzliche Beschichtungen zur Licht- und Wetterstabilisierung aufgebracht werden.

7. Verfahren nach Anspruch 1, **dadurch gekennzeichnet, dass** auf das umlaufende Metallband zunächst ein Releasecoat bestehend aus einem Lack, einem Salz, einer salzartigen Verbindung oder einem organischen Material aufgebracht wird.

## Claims

1. A method for the manufacture of a nacreous pigment, **characterized in that** first the backing is produced by vacuum evaporation coating of a circulating metal belt and, after release from the substrate, the backing particles thus produced are comminuted to a desired size, these backing particles subsequently being provided by wet coating with at least another layer.

2. A method according to claim 1, **characterized in that** the backing particles consist of silicon oxide, silicate, boron oxide, borates, aluminum oxide, aluminates, titanium boride (TiB₂) or mixtures thereof.

3. A method according to claim 1, **characterized in that** the backing particles comprise network formers or network modifiers and/or barium sulfate for surface smoothing and/or soluble or insoluble inorganic or organic colorants.

4. A method according to claim 1, **characterized in that** the layers applied by wet chemical deposition consist of oxides of the metals zirconium, chromium, titanium, iron, zinc, oxide hydrates of these metals, ferrotitanium, titanium suboxides or mixtures thereof.

5. A method according to claim 4, **characterized in that** the metal oxides are reduced.

6. A method according to claim 1, **characterized in that** additional coatings are applied for light stabilization and weathering resistance.

7. A method according to claim 1, **characterized in that** a release coat may be applied to the coating, consisting of a paint, a salt, a salty compound or an organic material.

## Revendications

1. Procédé de production d'un pigment interférentiel, **caractérisé en ce qu'**une couche de support est d'abord produite par vaporisation sous vide d'une bande métallique circulaire et **en ce que** les particules de couche de support ainsi produites, après avoir été détachées du support, sont réduites à une taille souhaitée, sachant que ces particules de couche de support sont ensuite dotées, par enrobage humide, d'au moins un revêtement supplémentaire.

2. Procédé selon la revendication 1, **caractérisé en ce que** la particule de couche de support est constituée de silice, de silicate, d'oxyde de bore, de borates, d'alumine, d'aluminates, de borure de titane (TiB₂) ou de mélanges de ceux-ci.

3. Procédé selon la revendication 1, **caractérisé en ce que** les particules de couche de support comprennent des formateurs de réseau ou des modificateurs de réseau et/ou du sulfate de baryum pour le lissage de la surface et/ou des colorants inorganiques ou organiques, solubles ou non solubles.

4. Procédé selon la revendication 1, **caractérisé en ce que** les couches appliquées par procédé chimique humide sont constituées d'oxydes métalliques de zircon, chrome, titane, fer, zinc, d'hydroxydes de ces métaux, de titanate de fer, de sous-oxydes de titane ou de mélanges de ceux-ci.

5. Procédé selon la revendication 4, **caractérisé en ce que** les oxydes métalliques sont réduits.

6. Procédé selon la revendication 1, **caractérisé en ce que** des revêtements supplémentaires sont appliqués pour assurer la stabilisation à la lumière et aux intempéries.

7. Procédé selon la revendication 1, **caractérisé en ce qu'**un revêtement anti-adhérent constitué d'une laque, d'un sel, d'un composé salin ou d'une matière organique est ensuite appliqué sur la bande métallique circulaire.
